# EUROPEAN PATENT APPLICATION

(11) **EP 4 516 236 A1**
(43) Date of publication of application: **05.03.2025**
(21) Application number: 23811952.3
(22) Date of filing: 09.03.2023
(51) Int. Cl.: A61B 8/00, A61B 8/12, A61B 1/00

(54) **ULTRASONIC PROBE DEVICE FOR ENDOSCOPY DEVICE**

(30) Priority: 26.05.2022 KR 20220064472; 23.12.2022 KR 20220183619
(71) Applicant: Endolfin Co., Ltd., Anyang-si, Gyeonggi-do 14059 (KR)
(72) Inventor: KIM, In Soo, Incheon 22858 (KR); KIM, Hang Jung, Yongin-si, Gyeonggi-do 16960 (KR)
(74) Representative: BCKIP Part mbB
(86) International application number: PCT/KR2023/003221
(87) International publication number: WO 2023/229165

(57) **Abstract**

The present invention relates to an ultrasonic probe device for an endoscopy device, the ultrasonic probe device being coupled to a leading end of a tube of an endoscopy device to generate ultrasonography information. The present invention provides an ultrasonic probe device for an endoscopy device, the ultrasonic probe device including a tube mounting part mounted at a leading end of a tube of an endoscopy device, and an ultrasonic module coupled to the tube mounting part and configured to generate ultrasonography information.

## Description

### [Technical Field]

The present invention relates to an ultrasonic probe device for an endoscopy device. More specifically, the present invention relates to an ultrasonic probe device for an endoscopy device, the ultrasonic probe device being coupled to a leading end of a tube of an endoscopy device to generate ultrasonography information.

### [Background Art]

General endoscopy may observe mucous membrane layers in a digestive track. However, the endoscopy has difficulty in observing a submucosal layer, a muscle layer, or the like below the mucous membrane in the digestive track. Ultrasonic endoscopy is utilized to cope with the difficulty.

However, devices and facilities for ultrasonic endoscopy are very expensive, and only general hospitals and medical centers are equipped with ultrasonic endoscopes, which degrades accessibility to medical care.

U.S. Patent No. 10363014 discloses an ultrasonic assembly attached to an endoscope. However, there is a problem in that a structure of the ultrasonic assembly is complicated, and a distance from a leading end of an endoscope tube to an ultrasonic module is relatively long. **In** addition, there is a problem in that an ultrasonic image or an optical image cannot be acquired in case that a needle is used.

### [Disclosure]

### [Technical Problem]

The present invention has been made in an effort to provide an ultrasonic probe device for an endoscopy device, the ultrasonic probe device being capable of being attached to or detached from a leading end of a tube of an endoscopy device and having a simple structure.

### [Technical Solution]

The present invention provides an ultrasonic probe device for an endoscopy device, the ultrasonic probe device including a tube mounting part mounted at a leading end of a tube of an endoscopy device, and an ultrasonic module coupled to the tube mounting part and configured to generate ultrasonography information.

In the embodiment, the tube mounting part may be configured to cover an outer portion of an end surface of the tube while having a shape in which the end surface of the tube is exposed.

In the embodiment, the ultrasonic module may protrude forward from an end surface of the tube.

In addition, the ultrasonic module may be formed to expose forward an objective lens, a nozzle, and a light guide provided on an end surface of the tube.

In the embodiment, the ultrasonic probe device according to the present invention may further include: an insert part provided on the tube mounting part or the ultrasonic module and inserted into a common channel of the tube.

In the embodiment, the insert part may be configured to close the common channel.

In the embodiment, the insert part may adjoin a part of an inner peripheral surface of the common channel and be smaller than an inner diameter of the common channel.

In the embodiment, the ultrasonic probe device may further include: a sleeve coupled to an end rim of the tube mounting part and an outer peripheral surface of the tube and configured to fix an end of the tube mounting part to the outer peripheral surface of the tube of the endoscopy device.

In the embodiment, the ultrasonic module may include: a transducer configured to generate an ultrasonic signal, receive a signal reflected by an organ, and generate the ultrasonography information; a reflector configured to reflect the ultrasonic signal and the reflected signal; and a motor configured to rotate the reflector.

In the embodiment, an internal space of the ultrasonic module may be filled with a medium for transmitting ultrasonic waves.

In the embodiment, a sealing member configured to seal the internal space may be provided on a motor main body of the motor or a driving shaft of the motor.

In the embodiment, a partition wall may be provided to divide the internal space, and driving power of the motor may be transmitted to the reflector by a magnetic force.

In the embodiment, the ultrasonic probe device may further include: an extension cable connected to the ultrasonic module and extending to the common channel of the tube.

The ultrasonic probe device according to the present invention may include: a main body including the tube mounting part and a main housing protruding from one side of the tube mounting part, in which a rotation driving part including the motor is accommodated in the main housing, in which an ultrasonic module body is coupled to an opening portion of the main housing, in which a reflector assembly including the reflector is accommodated in the ultrasonic module body, in which the transducer is provided at one side of the reflector assembly, and in which driving power of the rotation driving part is transmitted to the reflector assembly by a magnetic force so that the reflector assembly is rotatable.

In the embodiment, a through-hole may be formed in a bottom surface of the main housing, and a wire for operating the rotation driving part and the transducer may be extended through the through-hole.

In the embodiment, the rotation driving part may include the motor and a driving side magnet coupled to a driving shaft of the motor.

In addition, the rotation driving part may include a flexible substrate coupled to a terminal of the motor and having a motor driving wire for operating the motor.

In addition, the terminal may be coupled to the flexible substrate by soldering.

In addition, the flexible substrate may be bonded to a body of the motor by a bonding part.

In the embodiment, a magnetic force blocking part may be provided between the motor and the driving side magnet.

In the embodiment, the ultrasonic module body may have an insertion end coupled to the main housing.

In the embodiment, the ultrasonic module body may have a reflector assembly accommodation space that accommodates the reflector assembly, and a reflector assembly support shaft may be provided on a bottom surface of the reflector assembly accommodation space and support the reflector assembly so that the reflector assembly is rotatable.

In addition, the reflector assembly may have a driven side magnet coupled to a bottom surface of the reflector.

In addition, a transducer housing may be coupled to an upper end of the ultrasonic module body and accommodate the transducer.

In addition, a sealing member may be provided on a coupling portion of the transducer housing coupled to the ultrasonic module body.

In addition, the transducer may include a transducer wire, and a wire guide through which the transducer wire passes may be formed in the ultrasonic module body.

In addition, the ultrasonic probe device may further include: a cap coupled to the ultrasonic module body and configured to cover an upper portion of the transducer housing.

### [Advantageous Effects]

The present invention provides the disposable ultrasonic probe device capable of being attached to or detached from the leading end of the tube of the endoscopy device.

The ultrasonic probe device according to the present invention may acquire ultrasonography information on the digestive system by acquiring optical images without hindering the acquisition of optical images by the endoscopy device.

In addition, according to the ultrasonic probe device according to the present invention, the cable required for control and communication may be connected to the outside of the endoscopy device by using the common channel formed in the tube of the endoscopy device.

In addition, according to the embodiment of the present invention, it is possible to improve the operational stability of the constituent elements and the driving elements that constitute the ultrasonic probe device.

### [Description of Drawings]

FIG. 1 is a perspective view illustrating a state in which an ultrasonic probe device according to a first embodiment of the present invention is coupled to a leading end of an endoscope tube.
FIG. 2 is a rear perspective view of the ultrasonic probe device according to the first embodiment of the present invention.
FIG. 3 is a front view illustrating a state in which the ultrasonic probe device according to the first embodiment of the present invention is coupled to the leading end of the endoscope tube.
FIG. 4 is a cross-sectional view (cross-section taken along line A-A' in FIG. 2) of the ultrasonic probe device according to the first embodiment of the present invention, i.e., a view illustrating a first example of an ultrasonic module.
FIG. 5 is a cross-sectional view illustrating a state in which the ultrasonic probe device according to the first embodiment of the present invention is coupled to the leading end of the endoscope tube.
FIG. 6 is a cross-sectional view (cross-section taken along line A-A' in FIG. 2) of the ultrasonic probe device according to the first embodiment of the present invention, i.e., a view illustrating a second example of the ultrasonic module.
FIG. 7 is a view illustrating a sealing member provided on the ultrasonic module of the ultrasonic probe device illustrated in FIG. 6.
FIG. 8 is a cross-sectional view (cross-section taken along line A-A' in FIG. 2) of the ultrasonic probe device according to the first embodiment of the present invention, i.e., a view illustrating a third example of the ultrasonic module.
FIG. 9 is a cross-sectional view (cross-section taken along line A-A' in FIG. 2) of the ultrasonic probe device according to the first embodiment of the present invention, i.e., a view illustrating a fourth example of the ultrasonic module.
FIG. 10 is a perspective view of an ultrasonic probe device according to a second embodiment of the present invention.
FIG. 11 is a cross-sectional view (cross-section taken along line B-B' in FIG. 10) of a mounting member provided in the ultrasonic probe device according to the second embodiment of the present invention.
FIG. 12 is a perspective view illustrating a state in which an ultrasonic probe device according to a third embodiment of the present invention is coupled to a leading end of an endoscope tube.
FIG. 13 is a cross-sectional view illustrating a state in which the ultrasonic probe device according to the third embodiment of the present invention is coupled to the leading end of the endoscope tube.
FIG. 14 is a perspective view illustrating a state in which an ultrasonic probe device according to a fourth embodiment of the present invention is coupled to a leading end of an endoscope tube.
FIG. 15 is a cross-sectional view illustrating a state in which the ultrasonic probe device according to the fourth embodiment of the present invention is coupled to the leading end of the endoscope tube.
FIG. 16 is a cross-sectional view illustrating a state in which an ultrasonic probe device according to a fifth embodiment of the present invention is coupled to a leading end of an endoscope tube.
FIG. 17 is a cross-sectional view illustrating a state in which an ultrasonic probe device according to a sixth embodiment of the present invention is coupled to a leading end of an endoscope tube.
FIG. 18 is a perspective view illustrating a state in which an ultrasonic probe device according to a seventh embodiment of the present invention is coupled to a leading end of an endoscope tube.
FIG. 19 is a front view of the ultrasonic probe device according to the seventh embodiment of the present invention.
FIG. 20 is a cross-sectional view of the ultrasonic probe device according to the seventh embodiment of the present invention.
FIG. 21 is an exploded perspective view of the ultrasonic probe device according to the seventh embodiment of the present invention.
FIG. 22 is a top plan view illustrating a main body of the ultrasonic probe device according to the seventh embodiment of the present invention when viewed from above.
FIG. 23 is a view illustrating a process of assembling a rotation driving part of the ultrasonic probe device according to the seventh embodiment of the present invention.
FIG. 24 is a view illustrating another example of the rotation driving part of the ultrasonic probe device according to the seventh embodiment of the present invention.
FIG. 25 is a view illustrating a process of assembling an ultrasonic module of the ultrasonic probe device according to the seventh embodiment of the present invention.
FIG. 26 is a view illustrating a state in which the ultrasonic module is coupled to the main body of the ultrasonic probe device according to the seventh embodiment of the present invention.
FIG. 27 is a perspective view illustrating another example of the ultrasonic probe device according to the seventh embodiment of the present invention.
FIG. 28 is a perspective view illustrating a state in which the main body and a sleeve are separated in another example of the ultrasonic probe device according to the seventh embodiment of the present invention.

### [Best Mode]

Hereinafter, exemplary embodiments of the present invention will be described in detail with reference to the accompanying drawings. First, in assigning reference numerals to constituent elements of the respective drawings, it should be noted that the same constituent elements will be designated by the same reference numerals, if possible, even though the constituent elements are illustrated in different drawings. In addition, in the description of the present invention, the specific descriptions of publicly known related configurations or functions will be omitted when it is determined that the specific descriptions may obscure the subject matter of the present invention. Further, the exemplary embodiments of the present invention will be described below, but the technical spirit of the present invention is not limited thereto and may of course be modified and variously carried out by those skilled in the art.

In addition, the components of the ultrasonic probe device described in accordance with the following embodiments may be combined with one another.

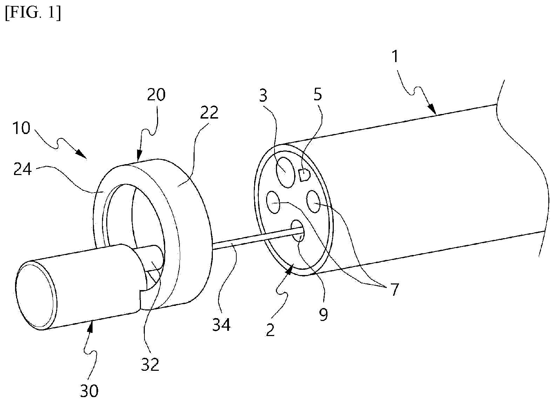

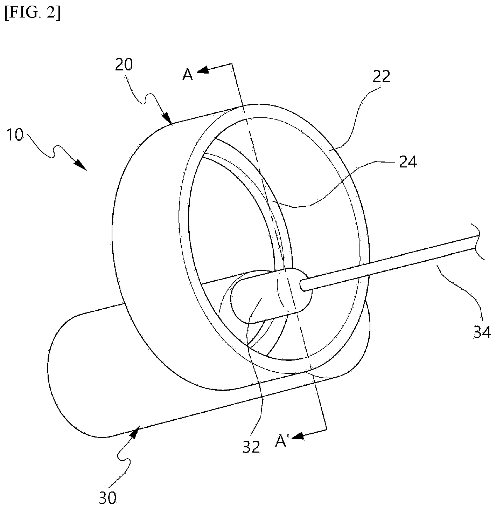

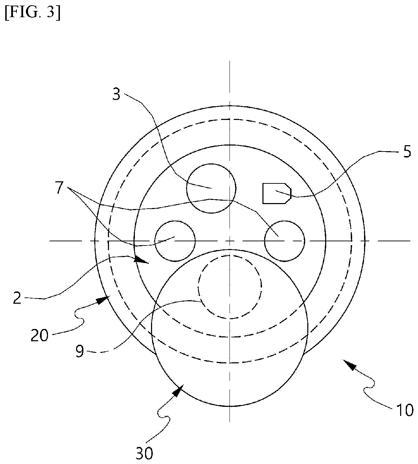

FIG. 1 is a perspective view illustrating a state in which an ultrasonic probe device according to a first embodiment of the present invention is coupled to a leading end of an endoscope tube, and FIG. 2 is a rear perspective view of the ultrasonic probe device according to the first embodiment of the present invention. In addition, FIG. 3 is a front view illustrating a state in which the ultrasonic probe device according to the first embodiment of the present invention is coupled to the leading end of the endoscope tube.

An ultrasonic probe device 10 according to a first embodiment of the present invention is coupled to a leading end of a tube 1 of an endoscope.

The tube 1 of the endoscope is made of a flexible material. An objective lens 3, which is configured to acquire an image, a nozzle 5, which is configured to spray air or water, a light guide 7, which is configured to emit light, and a common channel 9 are provided on an end surface 2 of the tube 1. The common channel 9 is also called a biopsy channel, an instrument channel, or the like, and the common channel 9 refers to a passageway through which instruments for biopsy and treatment are loaded or unloaded.

The ultrasonic probe device 10 includes a tube mounting part 20 mounted on a leading end portion of the tube 1, and an ultrasonic module 30 protruding forward from the tube mounting part 20. In addition, the ultrasonic probe device 10 may include an insert part 32 protruding rearward from the ultrasonic module 30 and inserted into the common channel 9 of the tube 1.

In the embodiment, the tube mounting part 20 may be configured to cover an outer peripheral surface of the tube 1 and an outer portion of the end surface 2 of the tube 1. In a state in which the tube mounting part 20 is coupled to the tube 1, the objective lens 3, the nozzle 5, the light guide 7, and the common channel 9 provided on the end surface 2 of the tube 1 are not covered. The tube mounting part 20 may include a ring-shaped annular portion 22 coupled to surround an outer peripheral surface of the leading end of the tube 1, and a stepped portion 24 extending from one end of the annular portion 22 and formed to cover a part of the outer portion of the end surface 2 of the tube 1.

The ultrasonic module 30 is coupled to the tube mounting part 20. The ultrasonic module 30 emits ultrasonic signals outward from the inside and receives ultrasonic waves reflected by human organs. The ultrasonic module 30 may be integrated with the tube mounting part 20. The ultrasonic module 30 may be disposed so as not to cover the objective lens 3, the nozzle 5, and the light guide 7 provided on the end surface 2 of the tube 1. Therefore, ultrasonography information may be acquired by the ultrasonic module 30, and optical images may be acquired through the objective lens 3.

The insert part 32 protruding from a rear surface of the ultrasonic module 30 is inserted into the common channel 9 of the tube 1. In the embodiment, the insert part 32 may have a shape that closes the common channel 9. An extension cable 34 is provided on the insert part 32 and exposed. The extension cable 34 may extend to the outside of a manipulation part of the endoscopy device through the common channel 9. That is, the extension cable 34 may extend to a manipulation part side opening of the tube 1 through the common channel 9. When the insert part 32 is configured to close the common channel 9, it is possible to prevent contamination in the common channel 9 during the endoscopy. In addition, because the extension cable 34 extends to the outside of the manipulation part of the endoscopy device through the common channel 9, a cable for the ultrasonic module may not be configured to be exposed to the outer peripheral surface of the tube 1. Meanwhile, in the embodiment of the present invention, the insert part 32 may be coupled to the tube mounting part 20.

In addition, in the embodiment of the present invention, the insert part 32 may not be provided, and the extension cable 34 extended from the ultrasonic module 30 may be connected to the outside through the common channel 9.

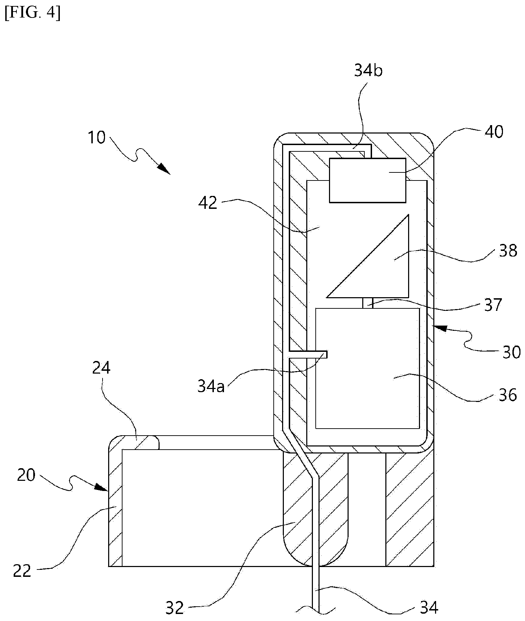

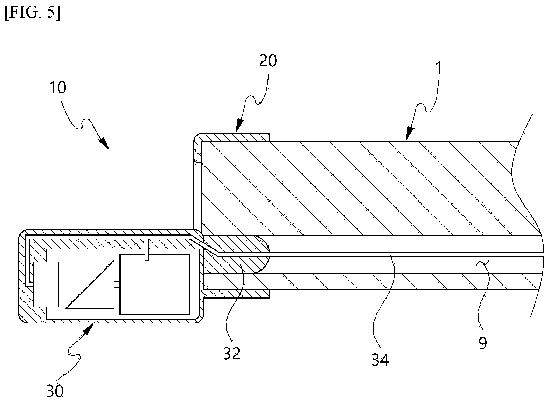

FIG. 4 is a cross-sectional view (cross-section taken along line A-A' in FIG. 2) of the ultrasonic probe device according to the first embodiment of the present invention, i.e., a view illustrating a first example of the ultrasonic module, and FIG. 5 is a cross-sectional view illustrating a state in which the ultrasonic probe device according to the first embodiment of the present invention is coupled to the leading end of the endoscope tube.

The ultrasonic module 30 may include a transducer 40 configured to generate an ultrasonic signal, receive a signal reflected by organs, and generate ultrasonography information, a reflector 38 configured to emit the ultrasonic signal, which is generated by the transducer 40, to an outer peripheral surface of the ultrasonic module 30 and transmit the signal, which is reflected by the organs, to the transducer 40, and a motor 36 configured to rotate the reflector 38. The reflector 38 is connected to a driving shaft 37 of the motor 36, such that the reflector 38 may rotate. Meanwhile, the extension cable 34 may include a motor control cable 34a provided for the motor 36, and a transducer cable 34b provided for the transducer 40.

The ultrasonic module 30 may be disposed in a shape approximately parallel to a central axis of the tube 1. When the reflector 38 rotates in a circumferential direction of the outer peripheral surface of the ultrasonic module 30, ultrasonography information on an inner wall of an organ may be acquired.

Meanwhile, an internal space 42 of the ultrasonic module 30, through which the ultrasonic waves pass, may be filled with a medium that makes it easy to transmit the ultrasonic waves. Oil, water-oil emulsion, aqueous gel, or the like may be used as the medium.

In the embodiment illustrated in FIG. 4, the configuration has been described in which ultrasonography information is acquired by the transducer 40, the reflector 38, and the motor 36. However, in the embodiment of the present invention, the configuration may be substituted with ultrasonic probes arranged in an array shape.

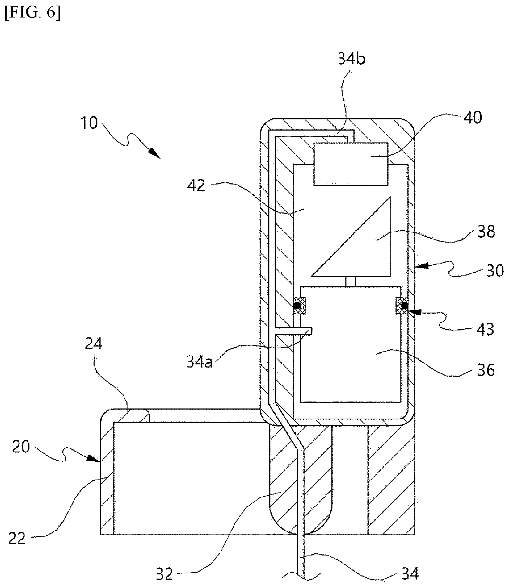

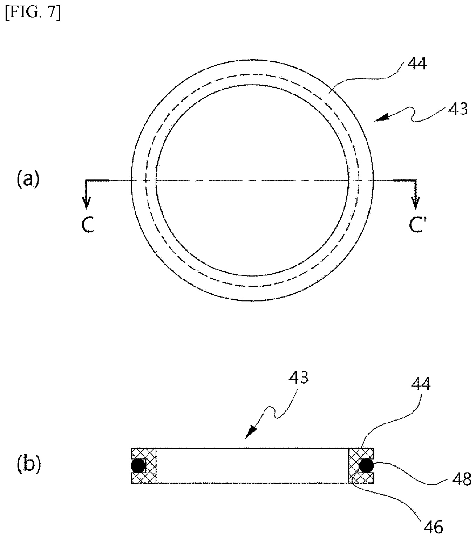

FIG. 6 is a cross-sectional view (cross-section taken along line A-A' in FIG. 2) of the ultrasonic probe device according to the first embodiment of the present invention, i.e., a view illustrating a second example of the ultrasonic module, and FIG. 7 is a view illustrating a sealing member provided on the ultrasonic module of the ultrasonic probe device illustrated in FIG. 6.

With reference to FIG. 6, a sealing member 43 may be provided between an outer peripheral surface of the motor 36 and an inner wall surface of the ultrasonic module 30 that defines the internal space 42. The sealing member 43 may prevent the medium, which is provided in the internal space 42 and serves to transmit ultrasonic waves, from leaking to a main body of the motor 36. With reference to FIG. 7, the sealing member 43 may include a ring-shaped sealing portion main body 44 configured to surround the motor 36, and an O-ring 48 fitted into a groove 46 formed in the sealing portion main body 44 in a transverse direction.

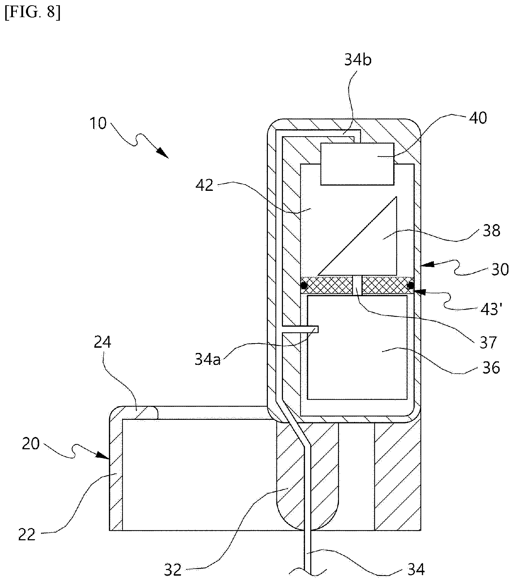

FIG. 8 is a cross-sectional view (cross-section taken along line A-A' in FIG. 2) of the ultrasonic probe device according to the first embodiment of the present invention, i.e., a view illustrating a third example of the ultrasonic module.

With reference to FIG. 8, a sealing member 43' may be provided to surround the driving shaft 37 of the motor 36.

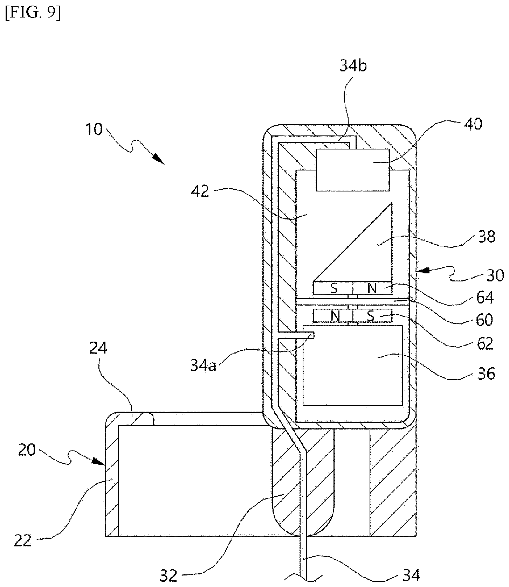

FIG. 9 is a cross-sectional view (cross-section taken along line A-A' in FIG. 2) of the ultrasonic probe device according to the first embodiment of the present invention, i.e., a view illustrating a fourth example of the ultrasonic module.

With reference to FIG. 9, the internal space of the ultrasonic module 30 may be divided by a partition wall 60, the motor 36 may be provided at one side of the partition wall 60, and the reflector 38 and the transducer 40 may be provided at the other side of the partition wall 60. The internal space 42 of the portion where the reflector 38 and the transducer 40 are positioned may be filled with a medium for transmitting ultrasonic waves.

A driving side magnet 62 is provided on a driving shaft of the motor 36, and a driven side magnet 64 is provided on a driven shaft of the reflector 38. When the driving side magnet 62 rotates, the driven side magnet 64 is rotated by a magnetic force, such that the reflector 38 may rotate.

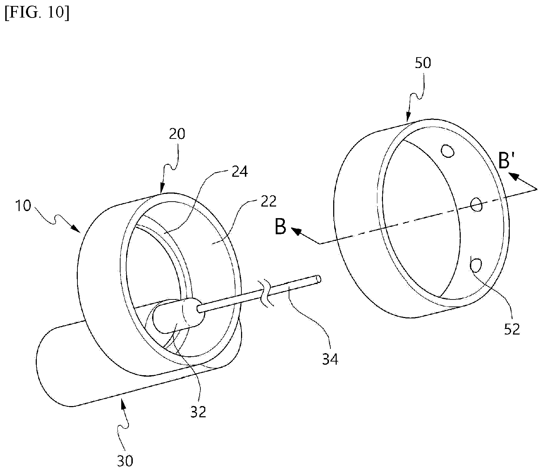

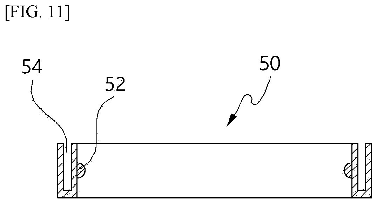

FIG. 10 is a perspective view of an ultrasonic probe device according to a second embodiment of the present invention, and FIG. 11 is a cross-sectional view (cross-section taken along line B-B' in FIG. 10) of a mounting member provided in the ultrasonic probe device according to the second embodiment of the present invention.

The ultrasonic probe device 10 according to the second embodiment of the present invention is characterized by further including a cover member 50. The cover member 50 may be made of a silicone material. The cover member 50 may be provided in a shape that surrounds the annular portion 22 of the tube mounting part 20. The cover member 50 may serve to provide a frictional force so that the probe device 10 is stably fixed to the outer peripheral surface of the tube 1. The cover member 50 may have a groove portion 54 into which the annular portion 22 is inserted. A protrusion 52 may be formed on an inner surface of the cover member 50 that is in contact with the tube 1.

The cover member 50 may be configured separately and mounted on the annular portion 22. Alternatively, the cover member 50 may be integrated with the annular portion 22 by insert molding.

However, in case that the annular portion 22 is made of a material, such as silicone, having flexibility and frictional force, the cover member 50 may not be provided separately.

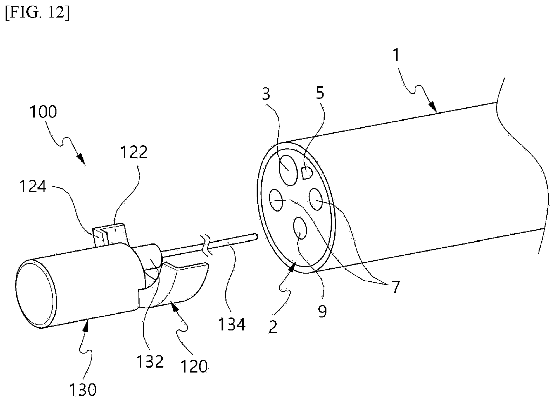

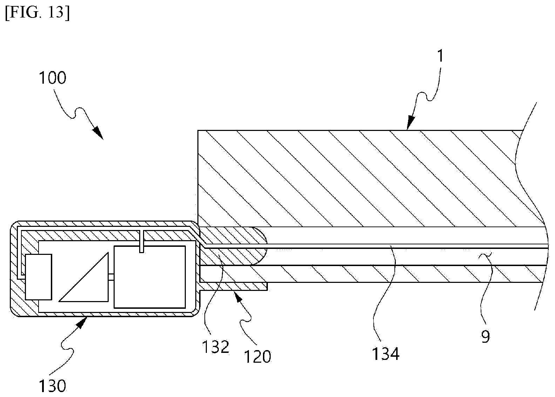

FIG. 12 is a perspective view illustrating a state in which an ultrasonic probe device according to a third embodiment of the present invention is coupled to a leading end of an endoscope tube, and FIG. 13 is a cross-sectional view illustrating a state in which the ultrasonic probe device according to the third embodiment of the present invention is coupled to the leading end of the endoscope tube.

An ultrasonic probe device 100 according to a third embodiment of the present invention includes a tube mounting part 120 configured to adjoin one side of the leading end of the tube 1, an ultrasonic module 130 protruding forward from the tube mounting part 120, and an insert part 132 protruding rearward from the ultrasonic module 130 and inserted into the common channel 9 of the tube 1. An extension cable 134 is exposed to the outside of the insert part 132. The configuration of the ultrasonic module 130 is identical to the configuration of the ultrasonic module 30 described in the first embodiment.

The tube mounting part 120 may include a tube outer portion support portion 122 configured to adjoin one side of the outer peripheral surface of the leading end of the tube 1, and a tube end surface support portion 124 extending from the tube outer portion support portion 122 and configured to adjoin the end surface 2 of the tube 1. The tube mounting part 120 is supported at one side of the leading end of the tube 1, and the insert part 132 is inserted into the common channel 9, such that the ultrasonic probe device 100 may be stably fixed to the leading end of the tube 1. A cover member may be further provided and surround the tube mounting part 120 in the same way as the cover member 50 illustrated in FIG. 10.

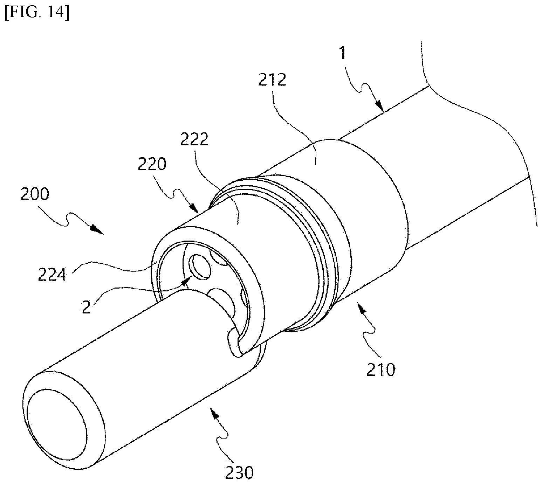

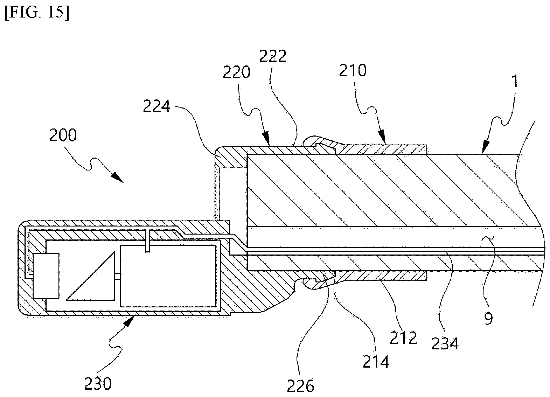

FIG. 14 is a perspective view illustrating a state in which an ultrasonic probe device according to a fourth embodiment of the present invention is coupled to a leading end of an endoscope tube, and FIG. 15 is a cross-sectional view illustrating a state in which the ultrasonic probe device according to the fourth embodiment of the present invention is coupled to the leading end of the endoscope tube.

An ultrasonic probe device 200 according to a fourth embodiment of the present invention includes a tube mounting part 220 configured to adjoin one side of the leading end of the tube 1, an ultrasonic module 230 protruding forward from the tube mounting part 220, and a sleeve 210 configured to support a coupled state of the tube mounting part 220 coupled to the leading end of the tube 1.

In the embodiment, the sleeve 210 may be tightly attached to the tube 1 and made of a material having flexibility. For example, the material may be silicone. Even in case that the tube mounting part 220 of the ultrasonic probe device 200 is made of a plastic material more rigid than a material of the sleeve 210, the ultrasonic probe device 200 may be assuredly fixed to the tube 1.

The tube mounting part 220 includes a ring-shaped annular portion 222 coupled to surround the outer peripheral surface of the leading end of the tube 1, and a stepped portion 224 extending from one end of the annular portion 222 and stepped to cover a part of the outer portion of the end surface 2 of the tube 1. In addition, the tube mounting part 220 has a fitting protrusion portion 226 extending from the other end of the annular portion 222.

The sleeve 210 includes a sleeve main body 212 having a cylindrical shape and mounted on the outer peripheral surface of the tube 1, and a fitting accommodation portion 214 formed at one side of the sleeve main body 212. The fitting protrusion portion 226 formed on the annular portion 222 of the ultrasonic probe device 200 is coupled to the fitting accommodation portion 214 of the sleeve 210, such that the ultrasonic probe device 200 is assuredly coupled to the tube 1.

Meanwhile, the position of the fitting protrusion portion 226 and the position of the fitting accommodation portion 214 may be changed, such that the fitting protrusion portion may be provided at one side of the sleeve main body, and the fitting accommodation portion may be provided at the other side of the annular portion.

In the fourth embodiment in FIGS. 14 and 15, unlike the first embodiment illustrated in FIG. 1, the insert part 32 is not provided, and an extension cable 234 extended from the ultrasonic module 230 is connected to the outside through the common channel 9. However, of course, in the embodiment of the present invention, the ultrasonic probe device 200 according to the fourth embodiment may be configured to have the insert part 32.

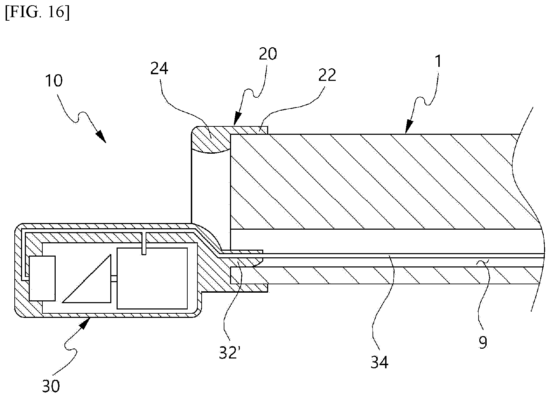

FIG. 16 is a cross-sectional view illustrating a state in which an ultrasonic probe device according to a fifth embodiment of the present invention is coupled to a leading end of an endoscope tube.

The basic configuration of the ultrasonic probe device 10 according to the fifth embodiment is identical to the configuration of the ultrasonic probe device 10 according to the first embodiment illustrated in FIG. 1. However, the fifth embodiment differs from the first embodiment in that the ultrasonic probe device 10 has an insert part 32' that is inserted into the common channel 9 but does not close the common channel 9. In addition, the fifth embodiment also partially differs from the first embodiment in that the stepped portion 24 is formed to cover the entire outer portion of the leading end surface of the tube 1.

In the embodiment, the insert part 32' may be in contact with a part of the inner peripheral surface of the common channel 9.

Because the insert part 32' does not close the common channel 9, it is possible to perform an operation of supplying a liquid, such as water, into the organ through the common channel 9.

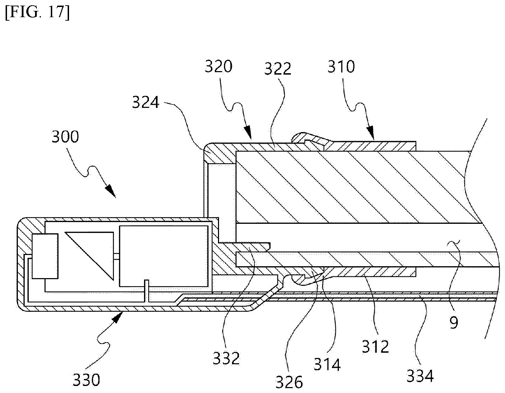

FIG. 17 is a cross-sectional view illustrating a state in which an ultrasonic probe device according to a sixth embodiment of the present invention is coupled to a leading end of an endoscope tube.

An ultrasonic probe device 300 according to a sixth embodiment of the present invention includes a tube mounting part 320 configured to adjoin one side of the leading end of the tube 1 and having an annular portion 322 and a stepped portion 324, an ultrasonic module 330 protruding forward from the tube mounting part 320, and a sleeve 310 configured to support a coupled state of the tube mounting part 320 coupled to the leading end of the tube 1. A fitting protrusion portion 326 may be provided on the annular portion 322, and the sleeve 310 may include a sleeve main body 312 and a fitting accommodation portion 314.

The tube mounting part 320 may further have an insert part 332 extending to the common channel 9 of the tube 1 and coupled to a part of an inner portion of the common channel 9.

The sixth embodiment differs from the above-mentioned embodiment in that an extension cable 334 connected to the ultrasonic module 330 extends toward the manipulation part of the endoscopy device along the outer portion of the tube 1 instead of extending into the common channel 9.

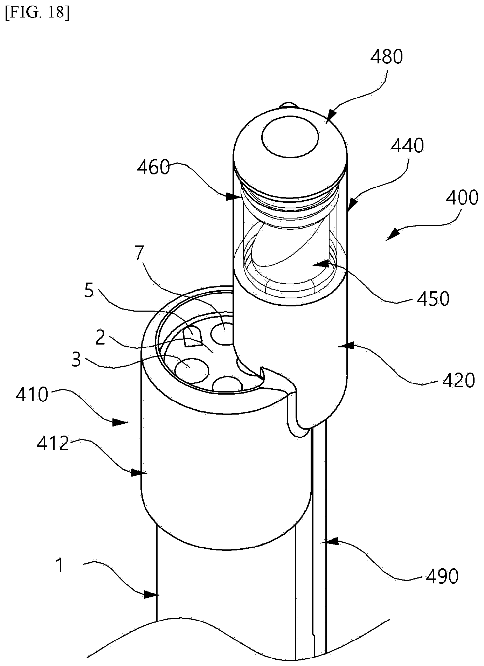

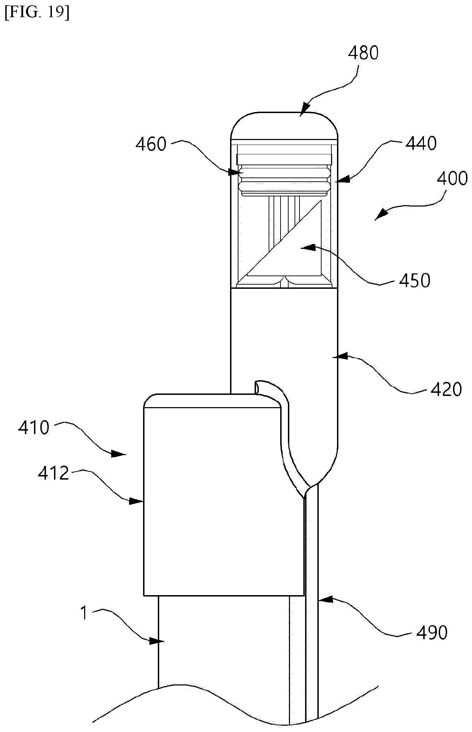

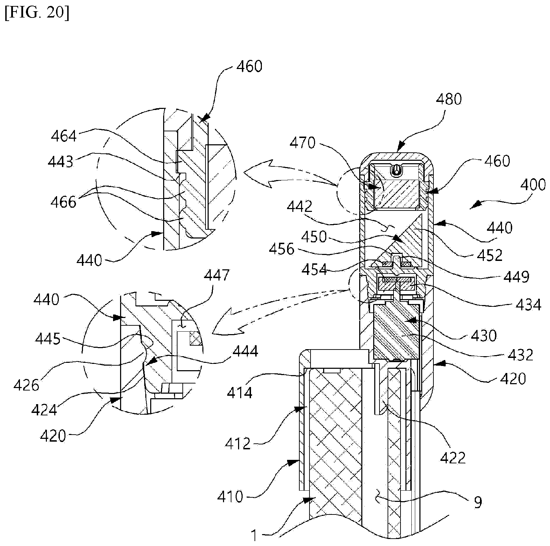

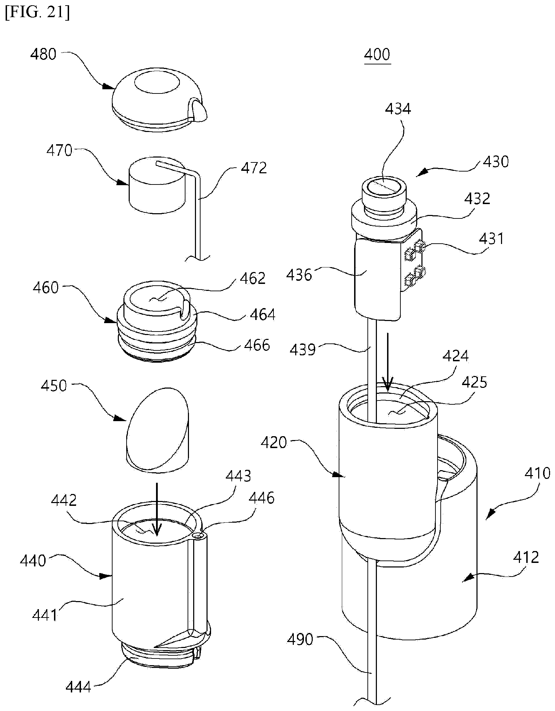

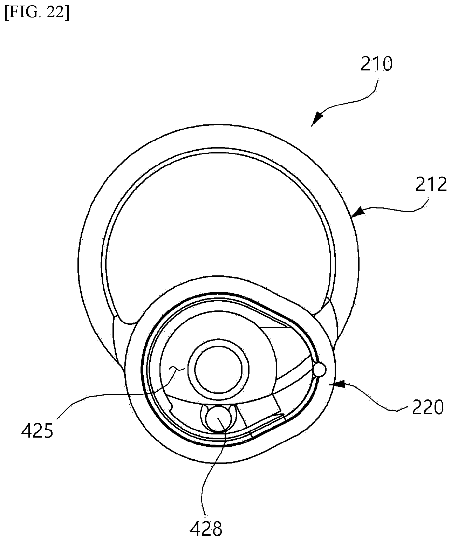

FIG. 18 is a perspective view illustrating a state in which an ultrasonic probe device according to a seventh embodiment of the present invention is coupled to a leading end of an endoscope tube, FIG. 19 is a front view of the ultrasonic probe device according to the seventh embodiment of the present invention, and FIG. 20 is a cross-sectional view of the ultrasonic probe device according to the seventh embodiment of the present invention. In addition, FIG. 21 is an exploded perspective view of the ultrasonic probe device according to the seventh embodiment of the present invention, and FIG. 22 is a top plan view illustrating a main body of the ultrasonic probe device according to the seventh embodiment of the present invention when viewed from above.

An ultrasonic probe device 400 according to a seventh embodiment of the present invention includes a main body 410 including a tube mounting part 412 mounted on the leading end portion of the tube 1, and an ultrasonic module coupled to the main body 410. The ultrasonic module is configured to be embedded in or coupled to the main body 410. The ultrasonic module may include a rotation driving part 430, an ultrasonic module body 440, a reflector assembly 450, a transducer housing 460, and a transducer 470. In addition, a cap 480 may be coupled to an upper end of the ultrasonic module body 440.

The main body 410 includes the tube mounting part 412 mounted on the leading end portion of the tube 1, and a main housing 420 protruding approximately forward from one side of the tube mounting part 412 (in an approximately forward direction of the tube 1 of the endoscope).

The tube mounting part 412 may be coupled to the end of the tube 1 and formed in a cylindrical shape corresponding to an external shape of the tube 1. In the state in which the tube mounting part 412 is coupled to the tube 1, at least one of the objective lens 3, the nozzle 5, and the light guide 7 provided on the end surface 2 of the tube 1 may be configured not to be covered. A catching projection 414 may be formed at an end of the tube mounting part 412 corresponding to the leading end of the tube 1 and allow the tube mounting part 412 to be stably coupled to the leading end of the tube 1.

The main housing 420 is coupled to one side of the tube mounting part 412 and formed to have an outer diameter smaller than an outer diameter of the main housing 420. Therefore, the structures for endoscopy, such as the objective lens 3, the nozzle 5, and the light guide 7, formed on the end surface 2 of the tube 1 are not covered by the main housing 420.

In the embodiment, the main housing 420 may be configured in a container shape including a driving part accommodation space 425 capable of accommodating the rotation driving part 430. Meanwhile, an insert part 422 protruding downward may be provided on a lower portion of the main housing 420. The insert part 422 may be inserted into a hole, e.g., the common channel 9 formed in the tube 1 of the endoscopy device. The tube mounting part 412 is coupled to the outer peripheral surface of the leading end of the tube 1, and the insert part 422 is inserted into the hole such as the common channel 9 formed in the tube 1, such that the ultrasonic probe device 400 may be stably coupled to the leading end of the tube 1.

An ultrasonic module coupling portion 424 may be formed in an opening portion of an upper end of the main housing 420.

The rotation driving part 430 includes a motor 432, and a driving side magnet 434 that is a permanent magnet coupled to a driving shaft 433 of the motor 432 and configured to rotate. In the embodiment, the driving side magnet 434 may be configured in a disc shape having a predetermined thickness, and the driving side magnet 434 may be coupled to the driving shaft 433 of the motor 432 in a state in which the driving side magnet 434 is mounted on a magnet tray 435.

In the embodiment, a flexible substrate 436 may be coupled to the motor 432 and connected to terminals 431 to which electric power or electrical signal for operating the motor 432 is transmitted. A motor driving wire 439 connected to the flexible substrate 436 is extended to the outside of the ultrasonic probe device 400 through an extension cable 490.

With reference to FIG. 22, a through-hole 428 is formed through a bottom surface of the main housing 420, and the extension cable 490 may be extended downward from the main housing 420 through the through-hole 428. The extension cable 490 is provided as a separate component denoted by the reference numeral. However, the extension cable 490 may, of course, be made by extending the motor driving wire 439.

The rotation driving part 430 is accommodated in the driving part accommodation space 425 of the main housing 420, and a lower end of the ultrasonic module body 440 is coupled to the ultrasonic module coupling portion 424 of the main housing 420.

The ultrasonic module body 440 may define a reflector assembly accommodation space 442 and be configured in an approximately cylindrical shape having a closed lower side and an open upper side. An insertion end 444 is formed at a lower end of the ultrasonic module body 440 and coupled to the ultrasonic module coupling portion 424 of the main housing 420. The ultrasonic module body 440 is made of a material that may transmit ultrasonic waves.

With reference to FIG. 20, a concave portion 445 is formed in the insertion end 444, and a convex portion 426 is formed on the ultrasonic module coupling portion 424, such that the ultrasonic module body 440 may not be easily separated in the state in which the ultrasonic module body 440 is coupled to the main housing 420. The concave portion 445 and the convex portion 426 may, of course, be changed and respectively provided on the ultrasonic module coupling portion 424 and the insertion end 444. Meanwhile, an internal space 447 of the insertion end 444 may be used as a space in which the driving side magnet 434 is accommodated.

A stepped portion 443 may be provided at an inner upper end of the reflector assembly accommodation space 442, and the stepped portion 443 serves to support a lower end rim of a mounting ring 464 of the transducer housing 460.

A wire guide 446 may be formed in a longitudinal direction in the ultrasonic module body 440, and a transducer wire 472 passes through the wire guide 446. In addition, as illustrated in FIG. 21, a reflector assembly support shaft 449 may protrude from a bottom surface of the reflector assembly accommodation space 442 of the ultrasonic module body 440.

The reflector assembly 450 is accommodated in the reflector assembly accommodation space 442 of the ultrasonic module body 440, and the reflector assembly 450 is rotatably supported by the reflector assembly support shaft 449.

The reflector assembly 450 includes a reflector 452 having an inclined surface and configured to reflect ultrasonic waves, which are generated by the transducer 470, toward the ultrasonic module body 440 and reflect ultrasonic waves, which are reflected from the outside, toward the transducer 470, and a driven side magnet 454 coupled to a bottom surface of the reflector 452. A support shaft insertion groove 456 is formed in a bottom surface of the reflector assembly 450, and the reflector assembly support shaft 449 is inserted into the support shaft insertion groove 456.

The reflector assembly accommodation space 442 of the ultrasonic module body 440, which accommodates the reflector assembly 450, may be filled with a medium for transmitting ultrasonic waves. In case that the reflector assembly accommodation space is filled with the ultrasonic wave transmission medium and the driving shaft 433 of the motor 432 is coupled directly to the reflector assembly 450, it may be difficult to implement sealing for preventing the ultrasonic wave transmission medium from leaking.

In order to cope with the difficulty, in the present invention, the motor 432 and the reflector assembly 450 are spatially separated, and the driving side magnet 434, which is coupled to the driving shaft 433 of the motor 432, and the driven side magnet 454, which is provided on the bottom surface of the reflector assembly 450, are connected by a magnetic force. Therefore, when the driving side magnet 434 rotates, the driven side magnet 454 is rotated by the magnetic force, such that the reflector 452 may rotate.

The transducer housing 460 is coupled to an upper end of the ultrasonic module body 440 and has a transducer accommodation portion 462 that accommodates the transducer 470. The mounting ring 464 is provided on an outer peripheral surface of the transducer housing 460, and sealing members 466 are provided at a lower end of the mounting ring 464. With reference to FIG. 20, the transducer housing 460 is coupled to the ultrasonic module body 440 so that a lower end of the mounting ring 464 is caught by the stepped portion 443 of the ultrasonic module body 440, and the sealability is maintained by the sealing members 466, which may prevent the ultrasonic wave transmission medium from leaking.

The transducer 470 is accommodated in the transducer housing 460, and the cap 480 is coupled to an upper portion of transducer housing 460. The transducer wire 472 connected to the transducer 470 may be extended to the outside of the transducer housing 460 and extended to the through-hole 428 formed in the main housing 420 through the wire guide 446.

The extension cable 490 may be configured by integrating the motor driving wire 439 and the transducer wire 472. In some instances, the extension cable 490 may be configured by extending and binding the motor driving wire 439 and the transducer wire 472.

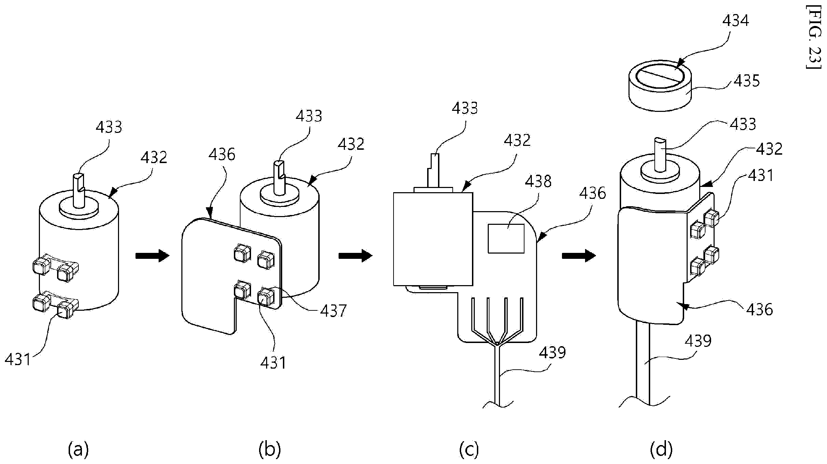

FIG. 23 is a view illustrating a process of assembling the rotation driving part of the ultrasonic probe device according to the seventh embodiment of the present invention.

With reference to part (a) of FIG. 23, the motor 432 has the driving shaft 433, and the terminals 431, to which electric power or electrical signal for operating the motor 432 is transmitted, are provided on the motor 432.

With reference to part (b) of FIG. 23, a terminal connection part 437 is coupled to the terminal 431 of the flexible substrate 436 by soldering or the like. The terminal 431 is formed on the motor 432, and the terminal connection part 437 of the flexible substrate 436 is connected to the terminal 431, such that a likelihood of disconnection of wires is minimized.

With reference to part (c) of FIG. 23, a bonding part 438 is provided on an inner surface of the flexible substrate 436, and the bonding part 438 is bonded to a body of the motor 432. The motor driving wire 439 electrically connected to the terminal 431 is provided on the flexible substrate 436.

With reference to part (d) of FIG. 23, the driving side magnet 434 is coupled to the driving shaft 433 of the motor 432 to which the flexible substrate 436 is coupled.

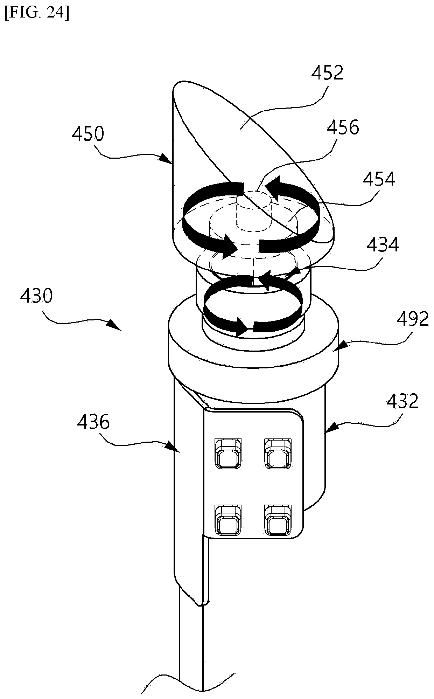

FIG. 24 is a view illustrating another example of the rotation driving part of the ultrasonic probe device according to the seventh embodiment of the present invention.

The driving side magnet 434, which is coupled to the motor 432 that constitutes the rotation driving part 430, is connected to the driven side magnet 454 of the reflector assembly 450 by a magnetic force and operates. A magnetic field formed by the driving side magnet 434 and the driven side magnet 454 may affect the magnetic force for operating the motor 432. In order to prevent the above-mentioned situation, a magnetic force blocking part 492 may be provided at an upper end of the motor 432, i.e., on a lower portion of the driving side magnet 434. In the embodiment, the magnetic force blocking part 492 may be made of carbon steel such as SM45C.

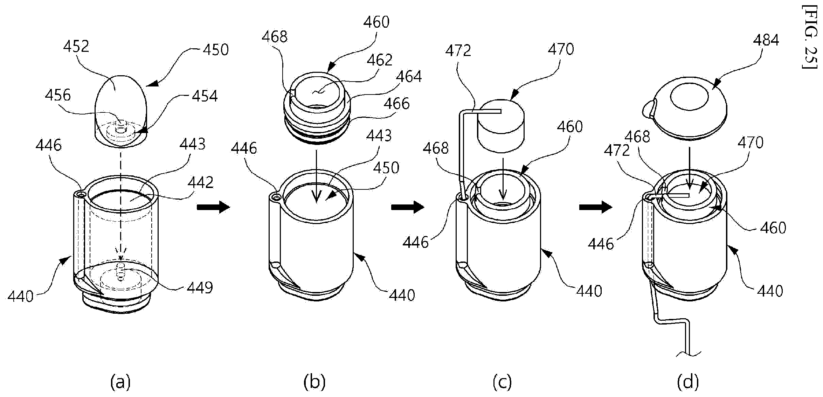

FIG. 25 is a view illustrating a process of assembling the ultrasonic module of the ultrasonic probe device according to the seventh embodiment of the present invention.

With reference to part (a) of FIG. 25, the reflector assembly 450 is coupled to the reflector assembly accommodation space 442 of the ultrasonic module body 440.

With reference to part (b) of FIG. 25, the transducer housing 460 is coupled to the upper end of the ultrasonic module body 440 to which the reflector assembly 450 is coupled. The transducer housing 460 may be coupled to the upper end of the ultrasonic module body 440 in a state in which the reflector assembly accommodation space 442 of the ultrasonic module body 440 is filled with the ultrasonic wave transmission medium or a state in which the ultrasonic module body 440 is immersed in a separate container that stores the ultrasonic wave transmission medium. In this case, the internal space of the ultrasonic module body 440 may be filled with the ultrasonic wave transmission medium without bubbles.

With reference to part (c) of FIG. 25, the transducer 470 is inserted into the transducer housing 460, the transducer wire 472 is extended through a wire extension groove 468 formed at an upper end of the transducer housing 460, and the transducer wire 472 passes through the wire guide 446.

With reference to part (d) of FIG. 25, the cap 480 is coupled to an opening portion provided at an upper end of the ultrasonic module body 440. The cap 480 may be provided to cover the opening portion provided at the upper side of the ultrasonic module body 440 and cover the upper portion of the wire guide 446.

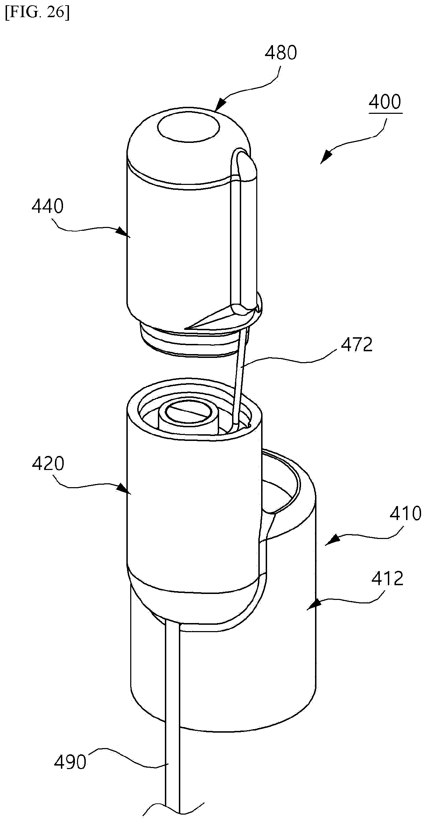

FIG. 26 is a view illustrating a state in which the ultrasonic module is coupled to the main body of the ultrasonic probe device according to the seventh embodiment of the present invention.

The ultrasonic module including the ultrasonic module body 440 is coupled to the upper end of the main housing 420 in the state in which the rotation driving part 430 is coupled to the main housing 420 of the main body 410. The transducer wire 472 may be extended through the through-hole 428 formed in the bottom surface of the main housing 420.

The ultrasonic probe device 400 according to the present invention may be coupled to the leading end of the tube 1 for endoscopy and acquire ultrasonic images.

The ultrasonic signal, which is generated by the transducer 470 provided in the ultrasonic module, is emitted outward by the reflector 452 that rotates, and the ultrasonic signal reflected by the human organs is reflected again by the reflector 452 and transmitted to the transducer 470.

The operation of the motor 432 and the generation and reception of the ultrasonic signal by the transducer 470 may be controlled by means of the extension cable 490 extending along the tube 1 to the outside of the human body.

According to the present invention, the ultrasonography may be performed by the ultrasonic probe device 400, and the optical image may be acquired through the objective lens 3 provided on the end surface 2 of the tube 1.

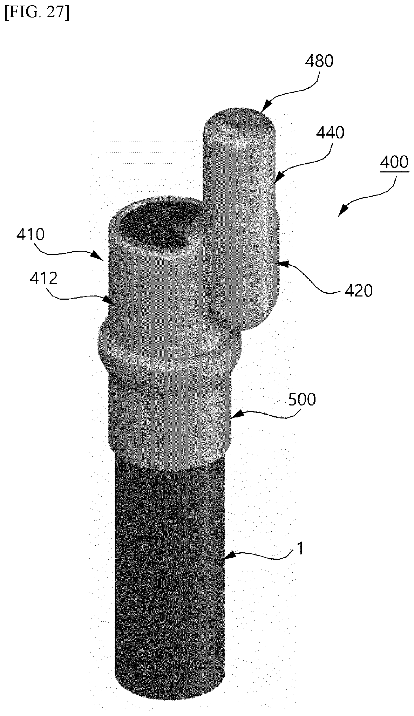

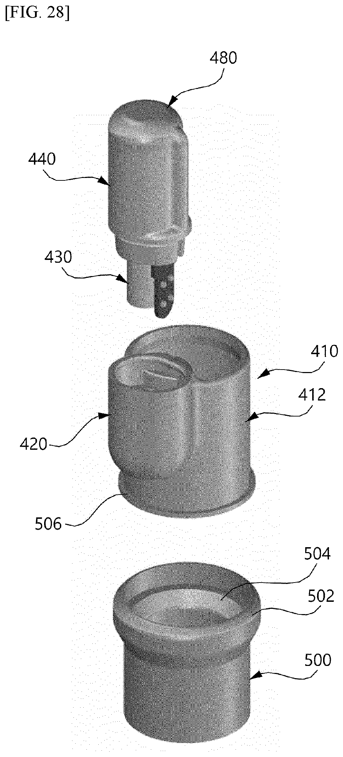

FIG. 27 is a perspective view illustrating another example of the ultrasonic probe device according to the seventh embodiment of the present invention, and FIG. 28 is a perspective view illustrating a state in which the main body and a sleeve are separated in another example of the ultrasonic probe device according to the seventh embodiment of the present invention.

With reference to FIGS. 27 and 28, a sleeve 500 may be further provided to assuredly fix the tube mounting part 412 of the main body 410 to the tube 1 of the endoscopy device. The sleeve 500 may be made of a material, such as silicone, having flexibility. In the embodiment, an anti-withdrawal protrusion 106 may be formed at a lower end of the tube mounting part 412, an overlapping portion 502 may be formed at an upper side of the sleeve 500 and surround an outer portion of the tube mounting part 412, and an anti-withdrawal groove 504 may be formed on an inner peripheral surface of the overlapping portion 502 so that the anti-withdrawal protrusion 506 is caught by the anti-withdrawal groove 504.

The configurations of the ultrasonic probe devices according to the first to seventh embodiments described above may be combined with one another, such that the ultrasonic probe device may be configured to be suitable for practical conditions in carrying out the present invention.

The above description is simply given for illustratively describing the technical spirit of the present invention, and those skilled in the art to which the present invention pertains will appreciate that various modifications, changes, and substitutions are possible without departing from the essential characteristic of the present invention. Accordingly, the embodiments disclosed in the present invention and the accompanying drawings are intended not to limit but to describe the technical spirit of the present invention, and the scope of the technical spirit of the present invention is not limited by the embodiments and the accompanying drawings. The protective scope of the present invention should be construed based on the following claims, and all the technical spirit in the equivalent scope thereto should be construed as falling within the scope of the present invention.

## Claims

1. An ultrasonic probe device comprising:
a tube mounting part configured to be mounted at a leading end of a tube of an endoscopy device; and
an ultrasonic module coupled to the tube mounting part and configured to generate ultrasonography information.

2. The ultrasonic probe device of claim 1, wherein the tube mounting part is configured to cover an outer portion of an end surface of the tube while having a shape in which the end surface of the tube is exposed.

3. The ultrasonic probe device of claim 1, wherein the ultrasonic module protrudes forward from an end surface of the tube.

4. The ultrasonic probe device of claim 3, wherein the ultrasonic module is formed to expose forward an objective lens, a nozzle, and a light guide provided on an end surface of the tube.

5. The ultrasonic probe device of claim 1, further comprising:
an insert part provided on the tube mounting part or the ultrasonic module and inserted into a common channel of the tube.

6. The ultrasonic probe device of claim 5, wherein the insert part is configured to close the common channel.

7. The ultrasonic probe device of claim 5, wherein the insert part adjoins a part of an inner peripheral surface of the common channel and is smaller than an inner diameter of the common channel.

8. The ultrasonic probe device of claim 1, further comprising:
a sleeve coupled to an end rim of the tube mounting part and an outer peripheral surface of the tube and configured to fix an end of the tube mounting part to the outer peripheral surface of the tube of the endoscopy device.

9. The ultrasonic probe device of any one of claims 1 to 8, wherein the ultrasonic module comprises:
a transducer configured to generate an ultrasonic signal, receive a signal reflected by an organ, and generate the ultrasonography information;
a reflector configured to reflect the ultrasonic signal and the reflected signal; and
a motor configured to rotate the reflector.

10. The ultrasonic probe device of claim 9, wherein an internal space of the ultrasonic module is filled with a medium for transmitting ultrasonic waves.

11. The ultrasonic probe device of claim 9, wherein a sealing member configured to seal the internal space is provided on a motor main body of the motor or a driving shaft of the motor.

12. The ultrasonic probe device of claim 9, wherein a partition wall is provided to divide the internal space, and driving power of the motor is transmitted to the reflector by a magnetic force.

13. The ultrasonic probe device of claim 9, further comprising:
an extension cable connected to the ultrasonic module and extending to the common channel of the tube.

14. The ultrasonic probe device of claim 9, comprising:
a main body comprising the tube mounting part and a main housing protruding from one side of the tube mounting part,
wherein a rotation driving part comprising the motor is accommodated in the main housing,
wherein an ultrasonic module body is coupled to an opening portion of the main housing,
wherein a reflector assembly comprising the reflector is accommodated in the ultrasonic module body,
wherein the transducer is provided at one side of the reflector assembly, and
wherein driving power of the rotation driving part is transmitted to the reflector assembly by a magnetic force so that the reflector assembly is rotatable.

15. The ultrasonic probe device of claim 14, wherein a through-hole is formed in a bottom surface of the main housing, and a wire for operating the rotation driving part and the transducer is extended through the through-hole.

16. The ultrasonic probe device of claim 14, wherein the rotation driving part comprises the motor and a driving side magnet coupled to a driving shaft of the motor.

17. The ultrasonic probe device of claim 16, wherein the rotation driving part comprises a flexible substrate coupled to a terminal of the motor and having a motor driving wire for operating the motor.

18. The ultrasonic probe device of claim 17, wherein the terminal is coupled to the flexible substrate by soldering.

19. The ultrasonic probe device of claim 17, wherein the flexible substrate is bonded to a body of the motor by a bonding part.

20. The ultrasonic probe device of claim 16, wherein a magnetic force blocking part is provided between the motor and the driving side magnet.

21. The ultrasonic probe device of claim 14, wherein the ultrasonic module body has an insertion end coupled to the main housing.

22. The ultrasonic probe device of claim 14, wherein the ultrasonic module body has a reflector assembly accommodation space that accommodates the reflector assembly, and
wherein a reflector assembly support shaft is provided on a bottom surface of the reflector assembly accommodation space and supports the reflector assembly so that the reflector assembly is rotatable.

23. The ultrasonic probe device of claim 22, wherein the reflector assembly has a driven side magnet coupled to a bottom surface of the reflector.

24. The ultrasonic probe device of claim 14, wherein a transducer housing is coupled to an upper end of the ultrasonic module body and accommodates the transducer.

25. The ultrasonic probe device of claim 24, wherein a sealing member is provided on a coupling portion of the transducer housing coupled to the ultrasonic module body.

26. The ultrasonic probe device of claim 24, wherein the transducer comprises a transducer wire, and a wire guide through which the transducer wire passes is formed in the ultrasonic module body.

27. The ultrasonic probe device of claim 24, further comprising:
a cap coupled to the ultrasonic module body and configured to cover an upper portion of the transducer housing.
